# EUROPEAN PATENT SPECIFICATION

(11) **EP 4 255 410 B1**
(45) Date of publication and mention of the grant of the patent: **06.11.2024**
(21) Application number: 21823571.1
(22) Date of filing: 03.12.2021
(51) Int. Cl.: A61K 31/20, A61K 31/40, A61K 45/06, A61P 13/12

(54) **SAROGLITAZAR AND MYRISTIC ACID FOR USE IN THE TREATMENT OF FOCAL SEGMENTAL GLOMERULOSCLEROSIS (FSGS)**
SAROGLITAZAR UND MYRISTINSÄURE ZUR ANWENDUNG BEI DER THERAPIE VON FOKAL SEGMENTALE GLOMERULOSKLEROSE (FSGS)
SAROGLITAZAR ET DE L'ACIDE MYRISTIQUE POUR UTILISATION DANS LE TRAITEMENT DE LA HYALINOSE SEGMENTAIRE ET FOCAL

(30) Priority: 03.12.2020 EP 20211662
(43) Date of publication of application: 11.10.2023
(73) Proprietor: Delta 4 GmbH, 1080 Vienna (AT)
(72) Inventor: PERCO, Paul, 1080 Vienna (AT); GEBESHUBER, Christoph, 1080 Vienna (AT)
(74) Representative: Loidl, Manuela Bettina
(86) International application number: PCT/EP2021/084254
(87) International publication number: WO 2022/117865

(56) References cited:
- WO-A1-2017/089979
- MAKLED MIRHAN N ET AL: "Saroglitazar attenuates renal fibrosis induced by unilateral ureteral obstruction via inhibiting TGF-[beta]/Smad signaling pathway", LIFE SCIENCE, PERGAMON PRESS, OXFORD, GB, vol. 253, 27 April 2020 (2020-04-27), XP086155237, ISSN: 0024-3205, [retrieved on 20200427], DOI: 10.1016/J.LFS.2020.117729
- KANJANABUCH T. ET AL: "PPAR-[gamma] agonist protects podocytes from injury", KIDNEY INTERNATIONAL, vol. 71, no. 12, 1 June 2007 (2007-06-01), GB, pages 1232 - 1239, XP055799122, ISSN: 0085-2538, DOI: 10.1038/sj.ki.5002248
- MONSERRAT ALBERTO J ET AL: "Protective effect of myristic acid on renal necrosis occurring in rats fed a methyl-deficient diet", RESEARCH IN EXPERIMENTAL MEDICINE, BERLIN, DE, vol. 199, no. 4, 1 February 2000 (2000-02-01), pages 195 - 206, XP002611868, ISSN: 0300-9130
- AVI Z. ROSENBERG ET AL: "Focal Segmental Glomerulosclerosis", CLINICAL JOURNAL OF THE AMERICAN SOCIETY OF NEPHROLOGY, vol. 12, no. 3, 27 February 2017 (2017-02-27), pages 502 - 517, XP055513283, ISSN: 1555-9041, DOI: 10.2215/CJN.05960616
- GEBESHUBER CHRISTOPH A ET AL: "Focal segmental glomerulosclerosis is induced by microRNA-193a and its downregulation of WT1", NATURE MEDICINE, NATURE PUB. CO, NEW YORK, vol. 19, no. 4, 17 March 2013 (2013-03-17), pages 481 - 487, XP036928700, ISSN: 1078-8956, [retrieved on 20130317], DOI: 10.1038/NM.3142

## Description

### FIELD OF THE INVENTION

The present invention relates to a pharmaceutical preparation comprising saroglitazar and myristic acid, or a salt or solvate thereof, for use in the treatment of focal segmental glomerulosclerosis (FSGS).

### BACKGROUND OF THE INVENTION

Focal segmental glomerulosclerosis (FSGS) is a severe glomerulopathy frequently leading to end stage kidney disease. FSGS is used to describe both a disease characterized by primary podocyte injury, and a lesion that occurs secondarily in any type of chronic kidney disease (CKD). It is defined by the presence of sclerosis in parts (segmental) or some glomeruli (focal) by light microscopy. FSGS can be found without an identifiable cause ("primary" or "idiopathic" FSGS represents 80% of cases) or in response to previous glomeruli injury, such as hypertension or obesity ("secondary" FSGS represents 20% of cases).

The most common manifestation of FSGS is proteinuria ranging from subnephrotic to nephrotic levels (heavy proteinuria, hypoalbuminemia and hyperlipidemia). Heavy proteinuria leads to progressive loss of kidney function (glomerulosclerosis) and kidney failure. It accounts for about 15% of end-stage renal disease (ESRD). Massive proteinuria (>10-15 g/day) leads to rapid deterioration of renal function and progression to ESRD within 2-3 years. The survival rate of FSGS patients with massive proteinuria is only 45%.

There is no approved drug for FSGS. The current standard of care for patients with FSGS include steroids, ACE inhibitors orARBs, immunosuppressive drugs such as glucocorticoids followed by calcineurin inhibitors, if needed, for intolerance or inadequate response to glucocorticoids; diuretics, plasmapheresis, diet change and statins.

WO2017/089979A1 discloses the use of Saroglitazar for the treatment of a disease or condition associated with abnormalities of the diabetic kidney.

Kanjanabuch T. et al. (2007) investigated the protection of podocytes from injury by the peroxisome proliferator-activated receptor-γ (PPAR-γ) agonist Pioglitazone.

Monserrat A. J. et al. (2000) described the protective effect of myristic acid on renal necrosis occurring in rats fed with a methyl-deficient diet.

Rosenberg A.Z. and Kopp J.B. (2017) refers to a review of focal segmental glomerulosclerosis (FSGS) which focusses on a practical approach to FSGS assessment on clinical and histopathological grounds in the context of the understanding of disease mechanisms and genetics.

There are, however, only poor response rates. Renin-angiotensin-aldosterone (RAAS) blockers are also used to control proteinuria, an important signature of FSGS. Existing treatments, however, achieved only limited success.

About 5400 patients are diagnosed with FSGS every year in the United States, but the number of cases is rising more than any other cause of Nephrotic Syndrome. Approximately 1,000 FSGS patients receive kidney transplants every year. Within hours to weeks after a kidney transplant, however, FSGS returns in approximately 30-40% of patients. Only 20% of patients, however, achieve complete remission after 5 years of treatment, and 40% of patients show no remission (Troyanov S. et al., 2005).

Despite best care, treatment failure is common and FSGS is causal in a significant proportion of end stage renal disease. Thus, an unmet need exists for novel disease modifying treatments for FSGS.

### SUMMARY OF THE INVENTION

The objective is solved by the subject of the present claims and as further described herein. It had been surprisingly found that the compounds of the present invention significantly lowered urine protein/creatinine ratio (UPCR) and urine albumin/creatinine ratio (UACR) in animal studies thus demonstrating significant functional improvement and reduced proteinuria.

The invention provides a pharmaceutical preparation comprising saroglitazar and myristic acid, or a salt or solvate thereof, for use in the treatment of focal segmental glomerulosclerosis (FSGS).

Specifically, FSGS is a recurrent focal segmental glomerulosclerosis, primary FSGS or native FSGS, specifically it is a glomerulopathy characterized by any one of heavy proteinuria, nephrotic syndrome, glomerulonephritis, membranoproliferative glomerulonephritis, IgA glomerulonephritis, progressive renal failure, or glomerular lesions on histopathology. Specifically, FSGS is not related to and is not caused by diabetes.

According to an embodiment of the invention, the pharmaceutical preparation is a medicinal product or a drug product, comprising saroglitazar and myristic acid, and a pharmaceutically acceptable carrier.

Specifically, the pharmaceutical preparation described herein is administered systemically.According to a further embodiment, saroglitazar which is present with myristic acid in the preparation described herein comprises the structure:

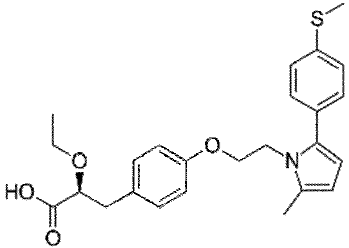

Specifically, the preparation comprises about 1 mg to 100 mg saroglitazar and about 1 mg to 1 g myristic acid.

According to a further embodiment, pharmaceutical preparation described herein is formulated for systemic administration, preferably for intravenous, intramuscular, subcutaneous, intradermal, transdermal, or oral administration.

According to a further embodiment, the pharmaceutical preparation described herein is administered to the subject as a spray, a powder, a gel, an ointment, a cream, a foam, or a liquid solution, a lotion, a gargle solution, an aerosolized powder, an aerosolized liquid formulation, granules, capsules, drops, tablet, syrup, lozenge, or a preparation for infusion or injection.

According to the invention the pharmaceutical preparation is for use in a subject is treated who suffers from FSGS or is at risk of developing FSGS.

In a further embodiment, the pharmaceutical preparation described herein is applied in an effective amount into a subject suffering from FSGS or being at risk of developing FSGS.

Specifically, the preparation is administered as the sole substance, or wherein treatment or preparation is combined with a further treatment or preparation with one or more active substances.

More specifically, the preparation is administered in combination with an active agent selected from the group consisting of antiviral drugs, anticoagulants, immune modulators, antibody preparations from human sources, monoclonal antibodies, intensive care medications, antihypertensive agents, statins, vasodilators, steroids, cytotoxic drugs, diuretics, non-steroidal anti-inflammatory drugs (NSAIDs), cholesterol or triglyceride reducing agents.

### FIGURES

Figure 1: FSGS was induced with Adriamycin (ADR) and evaluated regarding proteinuria at the indicated times (in days) after induction. UPCR = urinary protein creatinine ratio - a marker for proteinuria and kidney damage; grey boxes (ctrl) = control mice with FSGS; white boxes (Saroglitazar + Myristic acid) = mice with FSGS, treated with Saroglitazar + Myristic acid; Boxplots indicate the median (midline), inter-quartile range (box) and 95% confidence intervals (whiskers) in addition to individual data points (scatter). P-values of t-tests are indicated above the data for each time point.
Figure 2: FSGS was induced with Adriamycin (ADR) and evaluated regarding proteinuria at the indicated times (in days) after induction. UACR = urinary albumin creatinine ratio - a marker for proteinuria and kidney damage; grey boxes (ctrl) = control mice with FSGS; white boxes (Saroglitazar + Myristic acid) = mice with FSGS, treated with Saroglitazar + Myristic acid; Boxplots indicate the median (midline), inter-quartile range (box) and 95% confidence intervals (whiskers) in addition to individual data points (scatter). P-values of t-tests are indicated above the data for each time point.

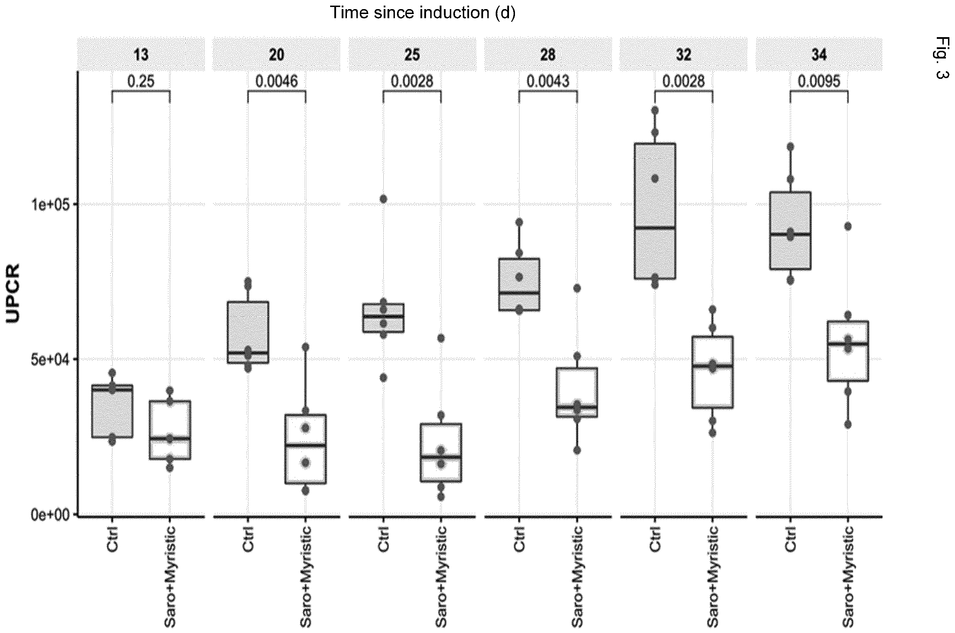

Figure 3: FSGS was induced by overexpression of miR-193a and resulting suppression of Wilms' tumor 1 (WT1) gene (Gebeshuber et al., 2013) and evaluated regarding proteinuria at the indicated times (in days) after induction. UPCR = urinary protein creatinine ratio - a marker for proteinuria and kidney damage; grey boxes (ctrl) = control mice with FSGS; boxes (marked Saro+Myristic) = mice with FSGS, treated with Saroglitazar + Myristic acid; Boxplots indicate the median (midline), inter-quartile range (box) and 95% confidence intervals (whiskers) in addition to individual data points (scatter). P-values of t-tests are indicated above the data for each time point.

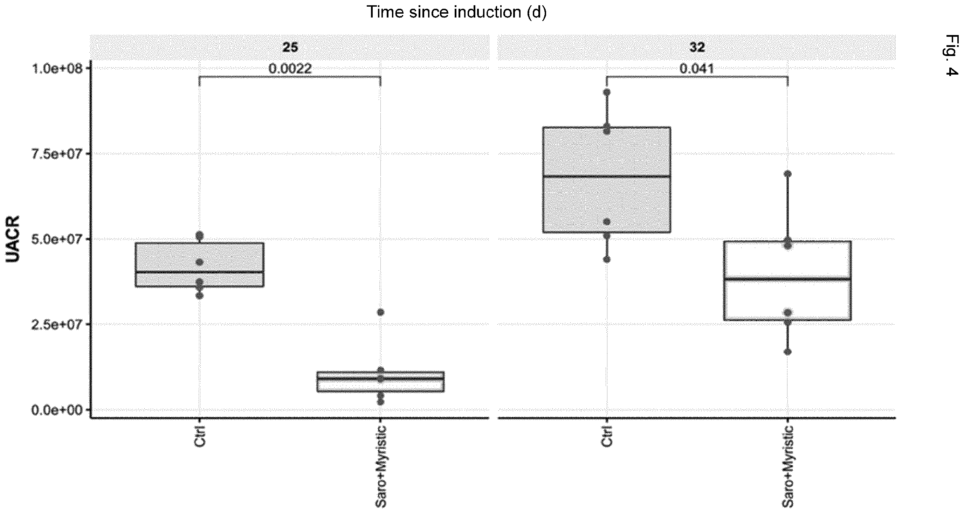

Figure 4: FSGS was induced by overexpression of miR-193a and resulting suppression of Wilms' tumor 1 (WT1) gene (Gebeshuber et al., 2013) and evaluated regarding proteinuria at the indicated times (in days) after induction. UACR = urinary albumin creatinine ratio - a marker for proteinuria and kidney damage; grey boxes (marked "ctrl2") = control mice with FSGS; boxes marked Saro+Myristic) = mice with FSGS, treated with Saroglitazar + Myristic acid; Boxplots indicate the median (midline), inter-quartile range (box) and 95% confidence intervals (whiskers) in addition to individual data points (scatter). P-values of t-tests are indicated above the data for each time point.
Figure 5: Experiment 1. UPCRs of all individual urines of control (Ctrl) and Saro- , Myr-, or Saro+Myr-treated animals after tail vein injection. p-values were calculated by Student's t-test and reveal significant improvement at multiple time points for Saro+Myr.
Figure 6: Experiment 2. UPCRs of all individual urines of control (Ctrl) and Saro+Myr-treated animals after tail vein injection. p-values were calculated by Student's t-test.
Figure 7: Experiment 3. UPCRs of all individual urines of control (Ctrl) and Saro+Myr-treated animals after induction of miR-193. p-values were calculated by Student's t-test and reveal significant improvement at multiple time points.
Figure 8: The mean reduction of UPCR due to Saro+Myr treatment is indicated over time. The mean UPCR reduction across all time points is indicated by the dotted line.
Figure 9: histological assessment with 20x magnification. Representative PAS stainings of adriamycin nephropathy control (upper left panel), adriamycin nephropathy Saro+Myr treated (upper right panel), miR-193a control (lower left panel) and adriamycin nephropathy Saro+Myr treated (lower right panel). Black arrows indicate sclerotic glomeruli. Black asterisks indicate protein casts representing tubular ectasia.
Figure 10: Histopathological effects of therapy. Average values of percentage of sclerotic glomeruli (left) and tubular ectasia stage (right) in the adriamycin nephropathy model are depicted. Error bars represent SEM (standard error of mean).
   Tubular ectasia scores: 0: none, 1: mild, 2: moderate, 3: strong
Fig. 11: Histopathological effects of therapy. Average values of percentage of sclerotic glomeruli (left) and tubular ectasia stage (right) in the miR-193a FSGS model are depicted. Error bars represent SEM (standard error of mean).
   Tubular ectasia scores: 0: none, 1: mild, 2: moderate, 3: strong

### DETAILED DESCRIPTION

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "about" as used herein refers to the same value or a value differing by +/-10% or +/-5% of the given value.

Saroglitazar as described herein may be used as a "physiologically acceptable salt". The choice of salt is determined primarily by how acid or basic the chemical is (the pH), the safety of the ionized form, the intended use of the drug, how the drug is given (for example, by mouth, injection, or on the skin), and the type of dosage form (such as tablet, capsule, or liquid).

Exemplary salts which are physiologically acceptable are sodium salts. However, it is also possible to employ, in place of the sodium salts, other physiologically acceptable salts, e.g., other alkali metal salts, alkaline earth metal salts, ammonium salts and substituted ammonium salts. The pharmaceutically acceptable salt of can be selected selected from organic or inorganic salts. Inorganic salt is selected from Calcium, Magnesium, Sodium, Potassium, Zinc, Ammonium and Lithium. Organic salt is selected from L- Arginine, Tromethamine, L-Lysine, Meglumine, Benethamine, Piperazine, Benzylamine, Dibenzylamine, Dicyclohexylamine, Diethylamine, Diphenylamine, α-naphthylamine, O- phenylenediamine, 1,3-Diaminopropane, (S)-a-naphthyl ethylamine, (S)-3- methoxyphenylethylamine, (S)-4-methoxyphenylethylamine, (S)-4-chlorophenyl ethylamine, (S)-4-methylphenylethylamine, Cinchonine, Cinchonidine, (-)-Quinine, Benzathine, Ethanolamine, Diethanol amine, Triethanolamine, imidazole, Diethylamine, Ethylenediamine, Choline, Epolamine, Morpholine 4-(2-hydroxyethyl), N-N-diethylethanolamine, Deanol, Hydrabamine, Betaine, Adamantanamine, L-Adamantanmethylamine and Tritylamine. Preferred substituted ammonium salts are those derived, for example, from lower mono-, di-, or trialkylamines, or mono-, di- and trialkanolamines. The free amino acids per se can also be used. Specific examples are ethylamine, ethylenediamine, diethylamine, or triethylamine salts.

The term "subject" or "patient" as used herein refers to a human being, or a non-human mammal, such as a dog, cat, horse, camelids, cattle or pig, suffering from FSGS or being at risk of developing FSGS. Specifically, it is a human being.

The term "non-diabetic subject" refers to a subject not suffering from Diabetes mellitus.

A subject "at risk" of developing FSGS may or may not have detectable disease or symptoms of disease, and may or may not have displayed detectable disease or symptoms of disease prior to the treatment methods described herein. "At risk" denotes that a subject has one or more risk factors, which are measurable parameters that correlate with development of FSGS, as described herein and known in the art. A subject having one or more of these risk factors has a higher probability of developing FSGS than a subject without one or more of these risk factor(s).

A "subject" can be a "patient" A "patient," refers to a "subject" who is under the care of a treating physician. In another embodiment, the patient is a subject who has not been diagnosed with FSGS. In yet other embodiments, the patient is a subject who has been diagnosed with FSGS but has not had any treatment to address the FSGS.

The term "pharmaceutically acceptable" also referred to as "pharmacologically acceptable" means compatible with the treatment of animals, in particular, humans. The term pharmacologically acceptable salt also includes both pharmacologically acceptable acid addition salts and pharmacologically acceptable basic addition salts.

The term" pharmacologically acceptable acid addition salt" as used herein means any non-toxic organic or inorganic salt of any base compound of the disclosure, or any of its intermediates. Basic compounds of the disclosure that may form an acid addition salt include, for example, compounds that contain a basic nitrogen atom. Illustrative inorganic acids which form suitable salts include hydrochloric, hydrobromic, sulfuric and phosphoric acids, as well as metal salts such as sodium monohydrogen orthophosphate and potassium hydrogen sulfate. Illustrative organic acids that form suitable salts include mono-, di-, and tricarboxylic acids such as glycolic, lactic, pyruvic, malonic, succinic, glutaric, fumaric, malic, tartaric, citric, ascorbic, maleic, benzoic, phenylacetic, cinnamic and salicylic acids, as well as sulfonic acids such as p-toluene sulfonic and methanesulfonic acids. Either mono-, di- or triacid salts can be formed, and such salts may exist in either a hydrated, solvated or substantially anhydrous form. In general, the acid addition salts of the compounds of the disclosure are more soluble in water and various hydrophilic organic solvents, and generally demonstrate higher melting points in comparison to their free base forms. The selection of the appropriate salt will be known to one skilled in the art. Other non-pharmacologically acceptable acid addition salts, e.g. oxalates, may be used, for example, in the isolation of the compounds of the disclosure, for laboratory use, or for subsequent conversion to a pharmacologically acceptable acid addition salt.

Specifically, saroglitazar may be present as saroglitazar magnesium salt.

Saroglitazar (S)-2-Ethoxy-3-(4-(2-(2-methyl-5-(4-(methylthio)phenyl)-1H-pyrrol-1-yl)ethoxy)phenyl)propanoic acid, (S)-alpha-ethoxy-4-[2-[-methyl-5-[4-(methylthio) phenyl]-1H-pyrrol-1-yl]ethoxy]benzene-propanoic acid. ((2S)-2-ethoxy-3-[4-(2- {2-methyl-5-[4-(methylsulfanyl)phenyl]-IH-pyrrol-I-yl}ethoxy)phenyl]propanoic acid) which is present together with myristic acid in the preparation has the structure

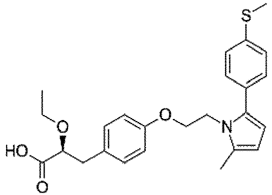

Saroglitazar is sold under the name Lipaglyn and is a novel first in class drug which acts as a dual PPAR agonist at the subtypes α (alpha) and γ (gamma) of the peroxisome proliferator-activated receptor (PPAR). Agonist action at PPARα lowers high blood triglycerides, and agonist action on PPARγ improves insulin resistance and consequently lowers blood sugar.

Saroglitazar is approved in India for the treatment of type 2 diabetes mellitus and dyslipidemia. Saroglitazar is indicated for the treatment of diabetic dyslipidemia and hypertriglyceridemia with type 2 diabetes mellitus not controlled by statin therapy.

Next to activating PPAR-α and PPAR-γ, saroglitazar was reported to have anti-inflammatory and anti-fibrotic effects via its regulatory effect on TGFB1/SMAD3 signaling (Makled M.N. et al., 2020). It also decreases renal levels NFKB1 and MMP9 (Makled M.N. et al., 2020). Saroglitazar was in addition shown to suppress Toll-like receptor 4 activation (Hassan N.F. et al., 2019).
Myristic acid is a long-chain fatty acid containing 14 carbon atoms. Myristic acid is a common saturated fatty acid with the molecular formula CH₃(CH₂)₁₂COOH. Its salts and esters are commonly referred to as myristates or tetradecanoates. It is named after the binomial name for nutmeg, from which it was first isolated. Myristic acid plays an important role in protein N-myristoylation, which is a cotranslational lipidic modification specific to the alpha amino group of an N-terminal glycine residue of many eukaryotic and viral proteins. The ubiquitous eukaryotic enzyme, N-myristoyltransferase, catalyzes the myristoylation process. Precisely, attachment of a myristoyl group increases specific protein-protein interactions leading to subcellular localization of myristoylated proteins with its signaling partners (Udenwobele D.I., et al., 2017).
According to the present invention, myristic acid belongs to the group of calcineurin inhibitors as it plays a role in myristoylation which, via the N-myristoyltransferase, regulates calcium sensitivity of calcineurin activation and prevents constitutive calcineurin activity.

According to an alternative embodiment, myristic acid may be replaced by one or more other calcineurin inhibitors such as, but not limited to tacrolimus, pimecrolimus, and cyclosporin.

According to a further embodiment, saroglitazar and myristic acid can also be combined with one or more calcineurin inhibitors known in the art.

Saroglitazar comprises about 1 to 100 mg saroglitazar per dose. Specifically, it can comprise 1, 2, 3, 4, 5, 6, 7, 8, 9, 10, 20, 30, 40, 50, 60, 70, 80, 90, 100, mg saroglitazar. It may be administered continuously at the same dose or as bolar administration at high dose, and further as reduced or low maintenance dose.

Myristic acid is present in combination with saroglitazar in an amount of about 1, 2, 3, 4, 5, 6, 7, 8, 9, 10, 20, 30, 40, 50, 60, 70, 80, 90, 100, 500, 1000 mg.

FSGS is a distinct clinico-pathological medical condition characterized by focal and segmental sclerosis in the kidney glomerulus and by podocyte foot process effacement (D'Agati V., 2003; Rosenberg A.Z. and Kopp J.B., 2017, Bose B. and Cattran D., 2014). The pathophysiology of FSGS can origin within the glomerulus ("primary FSGS") or be secondary to other reasons (e.g. hypertension; D'Agati V., 2003, Rosenberg A.Z. and Kopp J.B., 2017, Bose B. and Cattran D., 2014). Primary FSGS frequently leads to nephrotic syndrome characterised by proteinuria, hypoalbuminuria, hyperlipidemia and edema (D'Agati V., 2003; Rosenberg A.Z. and Kopp J.B., 2017, Bose B. and Cattran D., 2014). For a high number of cases the exact cause of FSGS is still elusive (idiopathic; D'Agati V., 2003; Rosenberg A.Z. and Kopp J.B., 2017, Bose B. and Cattran D., 2014). Known etiologies for FSGS are heterogeneous and include gene mutations, drugs, viruses, hypertension, and circulating factors - but not by diabetes (D'Agati V., 2003; Rosenberg A.Z. and Kopp J.B., 2017, Bose B. and Cattran D., 2014). Diabetic nephropathy - including diabetic glomerulosclerosis - represents the chronic loss of kidney function as a result of the pathophysiology of Diabetes mellitus (Qi et al., 2017).

Podocytes are atypical epithelial cells in the Bowman capsule in kidneys that wrap around capillaries of the glomerulus.

As used herein, FSGS is a scarring of glomeruli (sclerosis) and a damage to podocytes, specifically triggered by an endogenous factor that recognizes the podocyte as its target (podocytopathy, idiopathic or primary FSGS), or caused by an identifiable cause that indirectly injures podocytes (secondary FSGS), such as a genetic disorder, hypertension, inflammation, deregulation of proliferation, or mechanical stress.

The distinctive morphological picture of primary FSGS is a diffuse podocytopathy (+ at least one FSGS lesion) clinically dominated by a nephrotic syndrome, The diffuse podocyte toxicity involves almost all podocytes, with more than 50% of foot process effacement at the electron microscopy. A massive loss of albumin in the urine as well as hypoalbuminemia are commonly observed but are unusual in secondary forms even if nephrotic range proteinuria is present. Microvillous transformation, cytoplasm shedding, increased density of actin cytoskeleton on effaced foot processes, increased number of lysosomes and auto-phagocytic bodies are common features. Due to structural reorganization the risk of detachment of podocytes from the glomerular basement membrane (GBM) is increased, leaving wide areas of bare GBM. These "sticky" areas can produce adhesions, after which the deposition of hyaline material and mesangial matrix expansion progressively narrows the capillary lumen, until the affected segment is obstructed and sclerosed. Reversible glomerular prolapses into the proximal tubule are occasionally observed, and are the expression of an acute enlargement of the tuft and predictive of a 'tip lesion' (Angioi A., and Pani, A., 2016): Primary FSGS is part of the spectrum of podocytopathies that include minimal change disease (MCD).

Genetic forms of FSGS show similar alterations as described for primary FSGS.

Blood hypertension and adaptive mechanism can be major causes for secondary FSGS, both causing glomerular hypertension, accompanied by hypertrophied and stretched podocytes. Due to mechanical stress on the podocyte anchorage, the foot processes, structural reorganization of the cytoskeleton is generated, causing focal effacement.

The most aggressive form of FSGS is collapsing glomerulopathy, histologically shown by foot process effacement, segmental to global collapse of the capillary tuft, surrounded by a crown of hyperplastic podocytes (Angioi A., and Pani, A., 2016).

The symptoms of FSGS are defined by heavy proteinuria with optional biopsy confirmation of FSGS with glomerulosclerosis, glomerulonephritis (e.g. membranoproliferative glomerulonephritis (MPGN), IgA glomerulonephritis), nephrotic syndrome (hypoalbuminuria, hyperlipidemia, edema), progressive renal failure, glomerular lesions on histopathology, specifically as classified according to Haas M. et al., 2013, and podocyte fusion and injury.

FSGS can be diagnosed by methods well known in the art, such as, but not limited to determination of urinary protein /creatinine ratio (UPCR), urinary albumin / creatinine ratio (UACR), light microscopy of kidney biopsy, e.g. glomerular size, histologic variant of FSGS, microcystic tubular changes, and tubular hypertrophy; immunofluorescence, e.g. to rule out other primary glomerulopathies; and electron microscopy, e.g. extent of podocyte foot process effacement, podocyte microvillous transformation, and tubuloreticular inclusions.
Specifically, FSGS as used herein refers to non-diabetic renal disease. Specifically, diabetic nephropathy caused by Diabetes mellitus is excluded from the use of saroglitazar for the treatment of FSGS. Non-diabetic renal disease and diabetic renal disease can be confirmed and distinguished by methods known in the art such as biopsy.

In diabetes patients, nodular lesions and diffuse lesions are often observed. Classification methods for diabetic nephropathies are well known by the skilled person and are described by Qi C. et al., 2017, Tervaert T.W.C. et al., 2010, and Fioretto P. et al., 1996. Fioretto classification includes tubular, interstitial, and vascular lesions and divided diabetic nephropathies into 3 categories according to the pathological changes under light microscope: C1, normal/near normal; C2, typical diabetic nephropathy with predominantly glomerular changes; and C3, atypical patterns of injury, associated with disproportionately damage including tubulointerstitial or arteriolar hyalinosis and with absent or only mild diabetic glomerular changes. Tervaert pathological classification divides diabetic nephropathies into four classifications according to glomerular lesions, along with a separate scoring system for tubular, interstitial, and vascular lesions. Specifically, patients suffering from diabetes, specifically patients who are suffering from diabetes for more than 2 years, are excluded from being treated by saroglitazar as described herein.

Diabetic related nephropathies include diabetic retinopathy, diabetic nephropathy, and diabetes-related glomerulosclerosis.

The use of `FSGS' covers native FSGS, primary FSGS as well as recurrent FSGS, but specifically excluding diabetic FSGS.

Glomerulonephritis describes the inflammation of the membrane tissue in the kidney that serves as a filter, separating wastes and extra fluid from the blood.

MPGN is a form of glomerulonephritis caused by an abnormal immune response. Deposits of antibodies build up in a part of the kidneys called the glomerular basement membrane.

Glomerulosclerosis describes the scarring or hardening of the tiny blood vessels within the kidney. Although glomerulonephritis and glomerulosclerosis have different causes, they can both lead to kidney failure.

Nephrotic syndrome is a kidney disorder that causes the body to pass too much protein in urine. Nephrotic syndrome is usually caused by damage to the clusters of small blood vessels in kidneys.

As used herein, recurrent FSGS (rFSGS), or recurrence of FSGS is defined by heavy proteinuria with optional biopsy confirmation of FSGS with glomerular sclerosis and podocyte fusion and injury without evidence of acute rejection, glomerulitis or allograft glomerulopathy. As used herein, a recurrent FSGS (rFSGS) subject or patient is defined as someone who had FSGS prior to kidney transplant and then developed a recurrence of FSGS (rFSGS) following kidney transplant.

As used herein, a non-recurrent FSGS (nrFSGS) subject or patient is defined as someone has FSGS prior to kidney transplant but does not develop FSGS following kidney transplant.

As used herein, a native FSGS (nFSGS) subject or patient is defined as someone who has FSGS (heavy proteinuria with optional biopsy confirmation of FSGS with glomerular sclerosis and podocyte fusion and injury) in his own kidney prior to transplant.

The pharmaceutical preparation can be a medicinal product or a drug product, comprising saroglitazar and myristic acid, and a pharmaceutically acceptable carrier. The preparation described herein can also be used as food supplement.

Herein provided is also a method for treatment of FSGS wherein the pharmaceutical preparation comprises saroglitazar and myristic acid in an effective amount.

The term "effective amount" or "pharmaceutically effective amount" refers to that amount of compound that produces the desired effect for which it is administered (e.g., improvement in symptoms of FSGS, lessening the severity of FSGS or a symptom of FSGS, and/or reducing progression of FSGS or a symptom of FSGS). The exact amount of an effective dose will depend on the purpose of the treatment and will be ascertainable by one skilled in the art using known techniques (see, e.g., Lloyd V.A., 2016).

As used herein, the term "treatment" and its cognates refer to slowing or stopping disease progression. "Treatment" and its cognates as used herein, include, but are not limited to the following: complete or partial remission, lower risk of kidney failure (e.g. ESRD), and disease-related complications (e.g. edema, susceptibility to infections, or thrombo-embolic events). Improvements in or lessening the severity of any of these symptoms can be readily assessed according to methods and techniques known in the art or subsequently developed. Desirable effects of treatment include preventing the occurrence or recurrence of FSGS, specifically non-diabetic FSGS, or a condition or symptom thereof, alleviating a condition or symptom of FSGS, diminishing any direct or indirect pathological consequences of FSGS, decreasing the rate of FSGS progression or severity, and/or ameliorating or palliating the FSGS. In some embodiments, methods and compositions of the invention are used on patient sub-populations identified to be at risk of developing FSGS. In some cases, the methods and compositions of the invention are useful in attempts to delay development of FSGS.

In some embodiments, the compound of the pharmaceutical preparation described herein (i.e. a combination of saroglitazar with myristic acid), or a pharmaceutically acceptable salt or solvate thereof is not administered with any other therapeutic compound. In some embodiments, the compound is not administered with any other therapeutic compound, concurrently or sequentially. In some embodiments, the compound is administered alone.
In some embodiments, the method further comprises administering to the patient one or more additional therapeutic compound. In some embodiments, the one or more additional therapeutic compound is selected from one or more of an antiviral drug, an anticoagulant, an immune modulator, an antibody preparation from human sources, a monoclonal antibody, an intensive care medication, an antihypertensive agent, a statin, a vasodilator, an steroid, a cytotoxic drug, a diuretic, a non-steroidal anti-inflammatory drug (NSAID), a cholesterol or triglyceride reducing agent, PPAR agonists.

The at least one pharmaceutically acceptable carrier, as used herein, includes any and all solvents, diluents, other liquid vehicles, dispersion aids, suspension aids, surface active agents, isotonic agents, thickening agents, emulsifying agents, preservatives, solid binders, and lubricants, as suited to the particular dosage form desired. Remington: The Science and Practice of Pharmacy, 22nd edition, 2013, Pharmaceutical Press, and Encyclopedia of Pharmaceutical Technology, 2004, Taylor & Francis, disclose various carriers used in formulating pharmaceutical compositions and known techniques for the preparation thereof. Except insofar as any conventional carrier is incompatible with the compounds of this disclosure, such as by producing any undesirable biological effect or otherwise interacting in a deleterious manner with any other component(s) of the pharmaceutical preparation, its use is contemplated to be within the scope of this disclosure. Non-limiting examples of suitable pharmaceutically acceptable carriers include, but are not limited to, ion exchangers, alumina, aluminum stearate, lecithin, serum proteins (such as human serum albumin), buffer substances (such as phosphates, glycine, sorbic acid, and potassium sorbate), partial glyceride mixtures of saturated vegetable fatty acids, water, salts, and electrolytes (such as protamine sulfate, disodium hydrogen phosphate, potassium hydrogen phosphate, sodium chloride, and zinc salts), colloidal silica, magnesium trisilicate, polyvinyl pyrrolidone, polyacrylates, waxes, polyethylene-polyoxypropylene-block polymers, wool fat, sugars (such as lactose, glucose and sucrose), starches (such as corn starch and potato starch), cellulose and its derivatives (such as sodium carboxymethyl cellulose, ethyl cellulose and cellulose acetate, microcrystalline cellulose, methyl cellulose, sodium carboxymethyl cellulose), lactose, dextrin, mannitol, white sugar, maize starch, pre-gelatinized starch, precipitated calcium carbonate and calcium hydrogen phosphate,, powdered tragacanth, malt, gelatin, talc, excipients (such as cocoa butter and suppository waxes), oils (such as peanut oil, cottonseed oil, safflower oil, sesame oil, olive oil, corn oil and soybean oil), glycols (such as propylene glycol and polyethylene glycol), esters (such as ethyl oleate and ethyl laurate), agar, buffering agents (such as magnesium hydroxide and aluminum hydroxide), alginic acid, pyrogen-free water, isotonic saline, Ringer's solution, ethyl alcohol, phosphate buffer solutions, non-toxic compatible lubricants (such as sodium lauryl sulfate and magnesium stearate), coloring agents, releasing agents, coating agents, sweetening agents, flavoring agents, perfuming agents, preservatives, and antioxidants or any mixtures and combinations thereof.

### EXAMPLES

The foregoing description will be more fully understood with reference to the following examples. Such examples are, however, merely representative of methods of practicing one or more embodiments of the present invention and should not be read as limiting the scope of invention.

### Example 1

### In vivo Testing

For biological testing of the shortlisted compounds, we chose accepted mouse models of FSGS such as the adriamycin induced nephropathy or miR-193a-overexpressing mice which suffer from FSGS due to suppression of Wilms' tumor 1 (WT1) gene (Gebeshuber et al., 2013). All animal experiments and handling were in accordance with the Austrian law for protection of animals and approved by the animal ethics committee of the Austrian ministry for science and research.

Adriamycin-dependent nephropathy (active compound: doxorubicin) was induced by tail vein injection accepted doses of approx. 9 mg/kg mouse in 10 weeks old BALB/c mice. Control mice were injected with 0.9% saline. At least 6 mice were analyzed per group. Mice were monitored daily for signs of pain, altered movement, or reduced food uptake and sacrificed by cervical dislocation. Tested drugs were administered per oral administration via pre-mixed chow at drug doses based on literature research.

### Results

Urinary albumin levels were assessed by ELISA, urinary total protein and urinary creatinine levels were measured on the Roche Cobas system, using routine laboratory kits. Urinary protein/creatinine (UPCR) ratio and urinary albumin/creatinine ratio (UACR) were assessed at different time points. Weight and physical fitness scores were monitored on a daily basis. At the end of the experiment, tissue sections, plasma and urine samples were collected and stored until further analysis. For successful drug candidates significantly lowered UPCR and UACR values were found, demonstrating a functional improvement and reduced proteinuria.

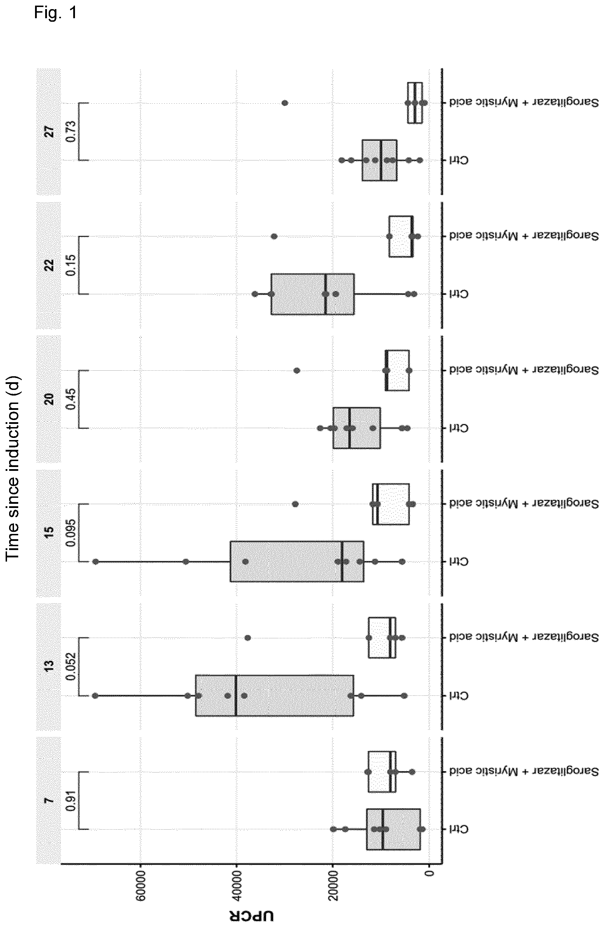

In the Adriamycin model, evaluating urinary protein creatinine ratio (UPCR), effects for the drug administration (Saroglitazar + Myristic acid) and timepoint (in days) were significant with p-values of 0.004 (drug) and 0.025 (timepoint), respectively. The p-value for the interaction (drug:timepoint) was 0.291 (see Figure 1).

In Fig. 1 it is shown that FSGS was induced with Adriamycin (ADR) and evaluated regarding proteinuria at the indicated times (in days) after induction. UPCR = urinary protein creatinine ratio - a marker for proteinuria and kidney damage; grey boxes (ctrl) = control mice with FSGS; white boxes (Saroglitazar + Myristic acid) = mice with FSGS, treated with Saroglitazar + Myristic acid; Boxplots indicate the median (midline), inter-quartile range (box) and 95% confidence intervals (whiskers) in addition to individual data points (scatter). P-values of t-tests are indicated above the data for each time point. Results for repeated measures ANOVA across timepoints are described above.

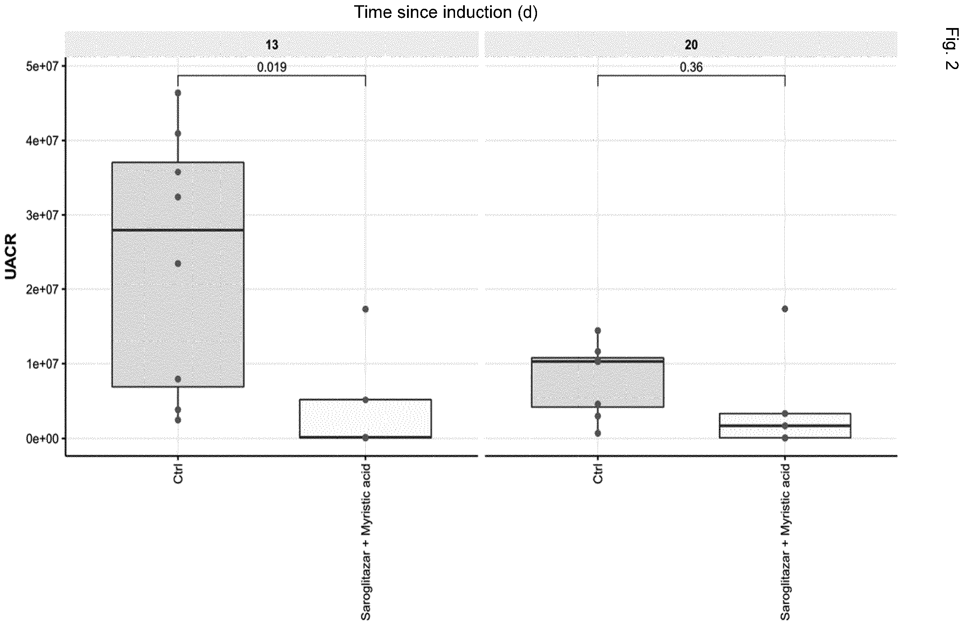

In the Adriamycin model, evaluating urinary albumin creatinine ratio (UACR), effects for the drug administration (Saroglitazar + Myristic acid) was significant with a p-value of 0.017 (drug). The p-values for the timepoint (in days) and the interaction were 0.088 (timepoint) and 0.090 (drug:timepoint), respectively (see Figure 2).

In Fig. 2 it is shown that FSGS was induced with Adriamycin (ADR) and evaluated regarding proteinuria at the indicated times (in days) after induction. UACR = urinary albumin creatinine ratio - a marker for proteinuria and kidney damage; grey boxes (ctrl) = control mice with FSGS; white boxes (Saroglitazar + Myristic acid) = mice with FSGS, treated with Saroglitazar + Myristic acid; Boxplots indicate the median (midline), inter-quartile range (box) and 95% confidence intervals (whiskers) in addition to individual data points (scatter). P-values of t-tests are indicated above the data for each time point. Results for repeated measures ANOVA across timepoints are described above.

In the miR193 model, evaluating urinary protein creatinine ratio (UPCR), effects for the drug administration (Saroglitazar + Myristic acid) and timepoint (in days) were significant with p-values of 2.21e-11 (drug) and 1.21e-07 (timepoint), respectively. The p-value for the interaction (drug:timepoint) was 0.121 (see Figure 3).

In Fig. 3 it is shown that FSGS was induced by overexpression of miR-193a and resulting suppression of Wilms' tumor 1 (WT1) gene (Gebeshuber et al., 2013) and evaluated regarding proteinuria at the indicated times (in days) after induction. UPCR = urinary protein creatinine ratio - a marker for proteinuria and kidney damage; grey boxes (ctrl) = control mice with FSGS; boxes (marked Saro+Myristic) = mice with FSGS, treated with Saroglitazar + Myristic acid; Boxplots indicate the median (midline), inter-quartile range (box) and 95% confidence intervals (whiskers) in addition to individual data points (scatter). P-values of t-tests are indicated above the data for each time point. Results for repeated measures ANOVA across timepoints are described above.

In the Adriamycin model, evaluating urinary albumin creatinine ratio (UACR), effects for the drug administration (Saroglitazar + Myristic acid) and timepoint (in days) were significant with p-values of 0.0001 (drug) and 0.0003 (timepoint), respectively. The p-value for the interaction (drug:timepoint) was 0.821 (see Figure 4).

In Fig. 4 it is shown that FSGS was induced by overexpression of miR-193a and resulting suppression of Wilms' tumor 1 (WT1) gene (Gebeshuber et al., 2013) and evaluated regarding proteinuria at the indicated times (in days) after induction. UACR = urinary albumin creatinine ratio - a marker for proteinuria and kidney damage; grey boxes (marked "ctrl2") = control mice with FSGS; boxes (marked Saro+Myristic) = mice with FSGS, treated with Saroglitazar + Myristic acid; Boxplots indicate the median (midline), inter-quartile range (box) and 95% confidence intervals (whiskers) in addition to individual data points (scatter). P-values of t-tests are indicated above the data for each time point. Results for repeated measures ANOVA across timepoints are described above.

### Example 2

### In vivo Testing

For biological testing of the shortlisted compounds, we chose accepted mouse models of FSGS such as the adriamycin induced nephropathy and miR-193a-overexpressing mice which suffer from FSGS due to suppression of Wilms' tumor 1 (WT1) gene (Gebeshuber et al., 2013). All animal experiments and handling were in accordance with the Austrian law for protection of animals and approved by the animal ethics committee of the Austrian ministry for science and research.

### Material & Methods

Adriamycin (Doxorubicin; Sigma, D1515-10mg) was diluted in ddH₂O. To induce FSGS, a dose of 10.5mg/kg mouse was injected i.v. into the tail vein using 27G needles, i. e. a total volume of 200 µl volume per 25 g mouse.

For tail vein injection, mice were anaesthetized using Ketanest (Pfizer, 25mg/ml vials) and Rompun (Bayer, 20mg/ml injection solution). A final mix of 12.5mg/ml Ketanest and 0.25% Rompun in 0.9% NaCl solution was prepared and 200µl/25g mouse were injected. For induction of miR-193a overexpression mice were fed with 1mg/ml Doxycycline hyclate (Sigma, D9891-10G) in 5% sucrose solution *ad libitum.* A combination of saroglitazar (via Sigma, from an Aldrich partner, ADVH7F37A3EA-100MG) and myristic acid (Sigma, M3128-10G) was used for therapy experiments.

### Animals/Animal maintenance

### Mice and FSGS model

Male Balb/c mice were obtained from the animal breeding facility of the Medical University (Abteilung für Labortierkunde, Himberg, Austria). MiR-193-overexpressing male mice in a Balb/c background were bred in the Medical University animal house. 16-22 weeks old animals with a weight >18g were used for the experiments and adjusted for weight and age. Animals were allowed acclimatization of at least 7 days. Animals were marked by ear or toe clip codes to allow identification.

### Diet

Standard commercial chow from ssniff (ssniff Spezialdiäten GmbH, Soest, Germany) contained crude protein (19%), crude fat (3.3%), crude fiber (4.9%), crude ash (6.4%), starch (36.5%), sugar (4.7%). Control group animals were fed standard chow, test group animals standard chow + saroglitazar (25mg/kg) + myristic acid (625mg/kg) produced by ssniff. All animals were allowed to eat *ad libitum* assuming a daily average uptake of ~4 grams/mouse (according to animal care guideline of the Johns Hopkins University, Baltimore, MD, USA; http://web.jhu.edu/animalcare/ procedures/mouse.html), i. e. ~4 mg saroglitazar/kg mouse/day (Jain et al. 2015) and ~100 mg myristic acid/kg mouse/day (Takato et al. 2017).

### Housing

The animals were kept in macrolon cages 375mm x 215mm x 150mm at standard conditions (23±1°C, 55±10% humidity, 12h day-night-cycle) and daily checked. Chow and water were provided *ad libitum.*

### Urine collection and measurements

Spot urine was collected every week and Creatinine and protein levels were measured.

Urinary albumin was measured by ELISA. 96-well plates were coated over night at 4°C with 100µl antibody solution Anti Mouse Serum Albumin antibody (Abcam ab34807, Lot GR3242102-4; diluted 1:2,000) in coating buffer (3.7g NaHCOs; 0,64g Na₂CO₃; 1L distilled water). The remaining procedure was performed at room temperature. Wells were washed 3x with HBSS (Sigma H8264-500ML) +0,05% Tween-20 (Bio-Rad, #1706531) and blocked in ELISA/ELISPOT Diluent (eBioscience, 00-4202-56) and washed again for 3x. Samples and standard curve were both diluted in ELISA/ELISPOT diluent were incubated for 2h, followed by 3 wash steps with HBSS+0,05% Tween-20, incubated with HRP antibody Anti Mouse Serum Albumin antibody (Abcam ab19195, Lot GR3242102-4; diluted 1:100,000) for 1h, followed by 5 wash steps. Reaction was developed with TMB One Solution (Promega, G7431) and stopped with 2N H₂SO₄. Standard curves were generated with Mouse Albumin (Merck/Sigma, 126674-25MG). 450nm absorbance was measured on an Epoch Microplate reader (BioTek) using Gen5 1.10 software.

### Histology

At the end of the experiment kidneys were removed, embedded in paraffin and PAS (Periodic acid-Schiff) staining was performed. Histology was thoroughly evaluated and the amount of sclerotic glomeruli and protein casts was quantified.

### Statistics

R version 4.0.2 was used for statistical analysis. Student's t-test was used for comparing UPCR and UACR values between samples from treated and control mice for each individual time point and to assess differences in histological parameters. Repeated measures ANOVA were performed to analyse the drug's impact on UPCR and UACR over time using the respective functions in the rstatix package (V0.6.0). ggplot2 (V3.3.2) and ggpubr (V0.4.0) packages were used for generating the graphical visualizations.

### Experimental setup

In three independent experiments, 51 Balb/c mice were either injected with 10.5mg/kg adriamycin into the tail vein or induced to overexpress miR-193a by doxycycline. Two days (adriamycin nephropathy) or 14 days (miR-193) post experiment start, respectively, the active group received standard chow supplemented with 25mg/kg saroglitazar and/or 625mg/kg myristic acid, while control mice stayed with standard chow. Spot urine was collected at least once per week, kidneys were harvested at sacrifice.

10.5mg/kg adriamycin were injected into the tail vein. After 2 days Saro+Myr chow therapy was started. Urine was collected and weight was measured weekly. The experiments lasted 4 weeks.

miR-193 overexpression was induced by 1mg/ml Doxycycline. After 14 days Saro+Myr chow therapy was started. At day 24, the Dox treatment was stopped. Urine was collected and weight was measured at least once per week. The experiments lasted 5 weeks.

In Table 1, an overview of the mice that were used in the experiments is provided (* 1 animal was found dead soon after the ADR injection).

**Table 1**

| | **E1 - ADR m** | | **E2 - ADR m** | | **E3 - 193 m** | |
|---|---|---|---|---|---|---|
| | started | completed | started | completed | started | completed |
| **Ctrl** | 7 | 7 | 7 | 7 | 6 | 6 |
| **Saro+Myr** | 6 | 6 | 6 | 5* | 6 | 6 |
| **Saro** | 7 | 7 | - | - | - | - |
| **Myr** | 6 | 6 | - | - | - | - |

### Results

### Saro+Myr therapy significantly reduces albuminuria and proteinuria

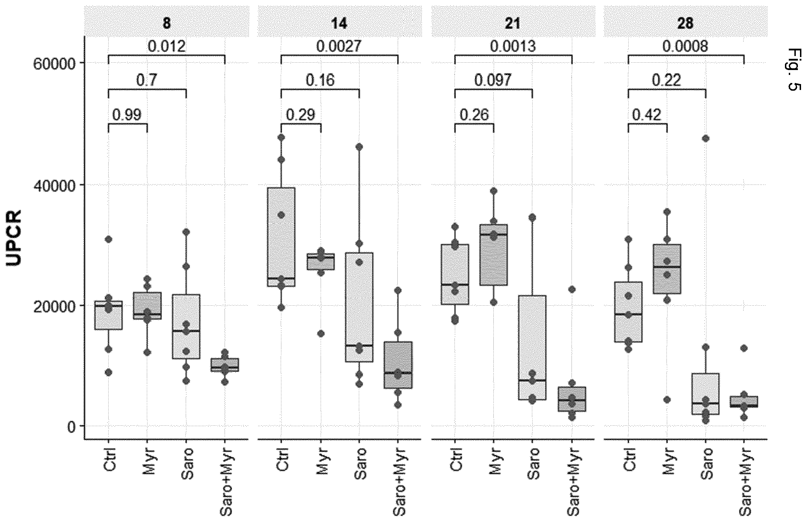

The impact of saroglitazar and myristic acid was determined either individually or in combination on FSGS in the adriamycin nephropathy model (Fig. 5). The level of proteinuria, the central clinical parameter for the diagnosis and therapy of FSGS, was determined by the urinary protein to creatinine ratio (UPCR).

As seen in Fig. 5, saroglitazar alone is able to ameliorate proteinuria to a certain extent, in particular at the two later time points. Myristic acid alone did not reduce UPCR. The strongest positive effect on proteinuria was however obtained with the combination of the two compounds leading to significant reductions in UPCR values across all time points.

Repeated measures ANOVA also showed the beneficial impact of Saro+Myr treatment over time on outcome determined based on UPCR (p=6.97e-10) in this experiment.

**ANOVA Table (type III tests) - UPCR**

| | Effect | DFn | DFd | F | p | p<.05 | ges |
|---|---|---|---|---|---|---|---|
| 1 | Experiment.drug | 1 | 44 | 61.389 | **6.97e-10** | * | 0.582 |
| 2 | Timepoint.days | 3 | 44 | 3.410 | 2.60e-02 | * | 0.189 |
| 3 | Experiment.drug:Timepoint.days | 3 | 44 | 1.478 | 2.34e-01 | | 0.092 |

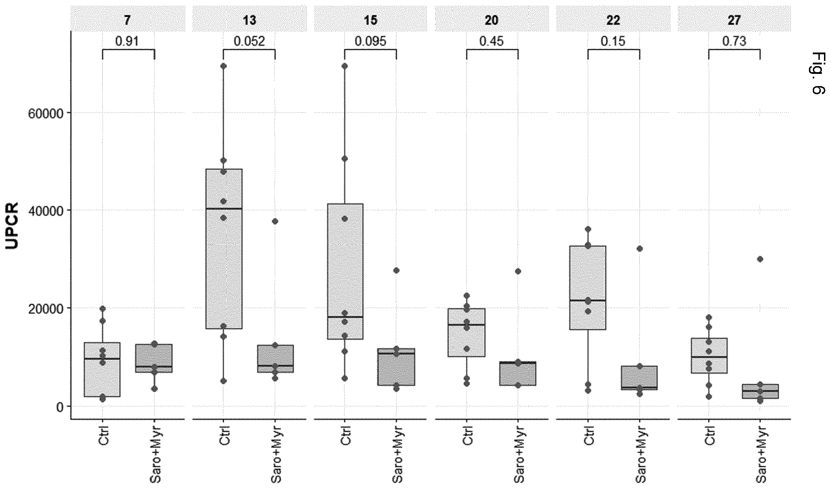

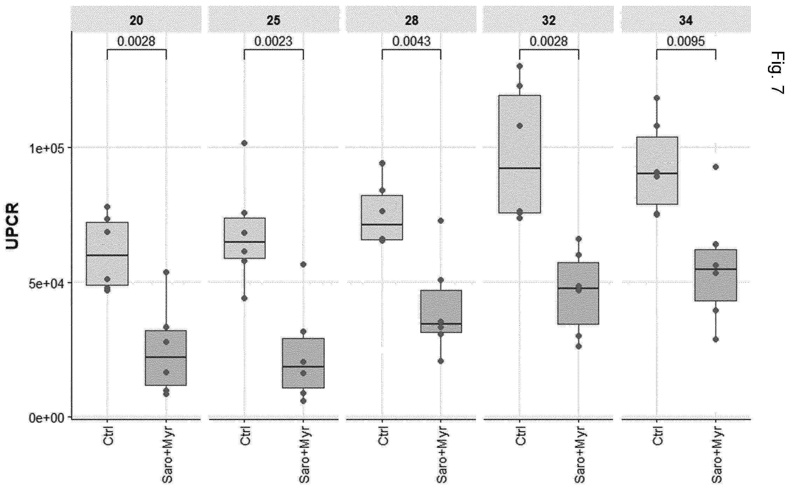

Saro+Myr therapy strongly and significantly ameliorated the most relevant clinical read-out parameter, i,e. proteinuria. Confirmation and analysis were shown in more detail by the positive impact of Saro+Myr in the adriamycin nephropathy model (Exp. 2, Fig. 6) and also in the miR-193a model (Exp. 3, Fig. 7).

Fig. 6 depicts box plots and p-values of all individual collected urines of experiment 2.

Repeated measures ANOVA revealed a significant beneficial impact of Saro+Myr treatment over time on outcome also in experiment 2 determined based on UPCR (p=0.004).

**ANOVA Table (type III tests)- UPCR**

| | Effect | DFn | DFd | F | p | p<.05 | ges |
|---|---|---|---|---|---|---|---|
| 1 | Experiment.drug | 1 | 66 | 9.109 | **0.004** | * | 0.121 |
| 2 | Timepoint.days | 5 | 66 | 2.762 | 0.025 | * | 0.173 |
| 3 | Experiment.drug:Timepoint.days | 5 | 66 | 1.261 | 0.291 | | 0.087 |

Fig. 7 depicts box plots and p-values of all individual collected urines of experiment 3.

Repeated measures ANOVA also showed the beneficial impact of Saro+Myr treatment over time on outcome also in experiment 3 determined based on UPCR (p=2.23e-11).

**ANOVA Table (type II tests)- UPCR**

| | Effect | DFn | DFd | F | p | p<.05 | ges |
|---|---|---|---|---|---|---|---|
| 1 | Experiment.drug | 1 | 50 | 73.416 | **2.23e-11** | * | 0.595 |
| 2 | Timepoint.days | 4 | 50 | 7.377 | 9.46e-05 | * | 0.371 |
| 3 | Experiment.drug:Timepoint.days | 4 | 50 | 0.457 | 7.67e-01 | | 0.035 |

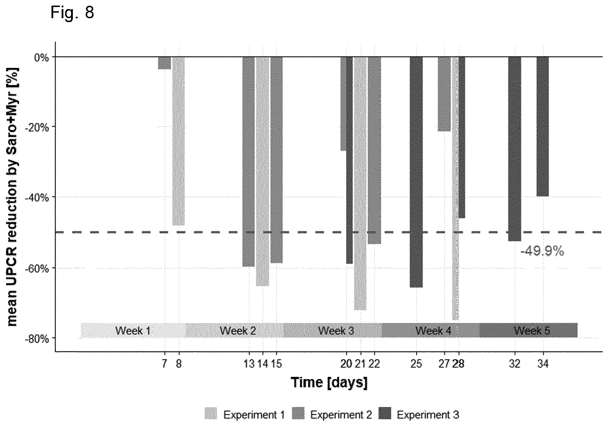

The beneficial impact of Saro+Myr therapy was thus confirmed in three independent experiments. Fig. 8 summarizes the effect of experiments 1-3 and depicts the average percent reductions of UPCR.

Taken all experiments together, Saro+Myr therapy revealed a marked and significant protection of renal function and reduced UPCR by 49.9% on average across all time points (repeated measures ANOVA p=5.00e-20). This is highly advantageous as an amelioration of UPCR is the primary outcome goal in clinical trials and slows down FSGS progression.

**ANOVA Table (type III tests)- UPCR**

| | Effect | DFn | DFd | F | p | p<.05 | ges |
|---|---|---|---|---|---|---|---|
| 1 | Experiment.drug | 1 | 160 | 110.865 | **5.00e-20** | * | 0.409 |
| 2 | Exp.Time | 14 | 160 | 32.801 | 7.71e-40 | * | 0.742 |
| 3 | Experiment.drug:Exp.Time | 14 | 160 | 3.995 | 6.85e-06 | * | 0.259 |

### Weight change

We evaluated the weight change weekly over the course of the experiment. The average weight reduction in the control group across all timepoints and experiments as compared to the starting weight was 10.2% for the control and 11.8% for saroglitazar and myristic acid.

### Histopathological assessment

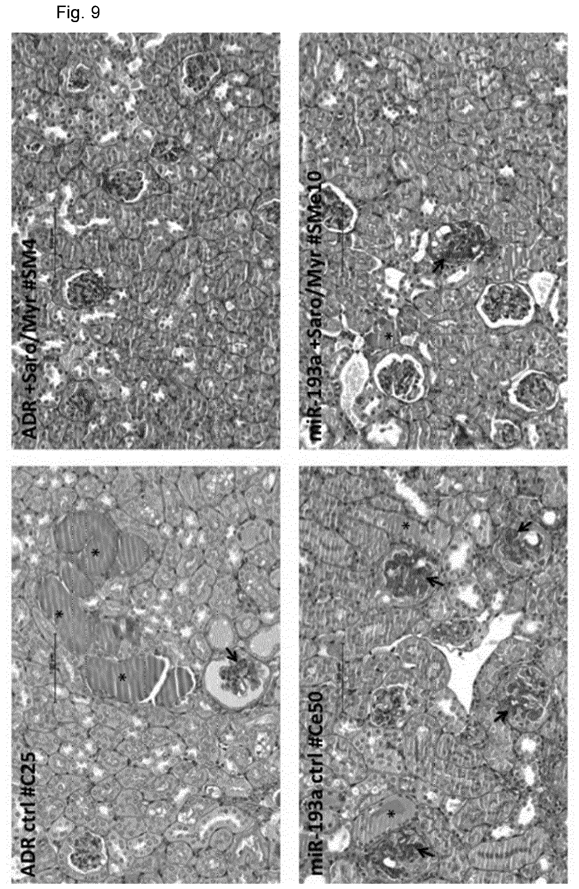

The impact of Saro+Myr on pathologic lesions in FSGS as an accompanying parameter to the strong amelioration of proteinuria was evaluated (Fig. 9-11).

Fig. 9 depicts the strongest affected samples of the adriamycin nephropathy model and the miR-193a model, age-matched samples were selected. For both models, Saro+Myr treatment ameliorated severe disease and reduced the amount of sclerotic glomeruli and tubular ectasia. Tubular ectasia, determined by protein cylinders in PAS staining observed as uniform areas in the picture, is an alternate measure to identify a failure in the renal filtration process.

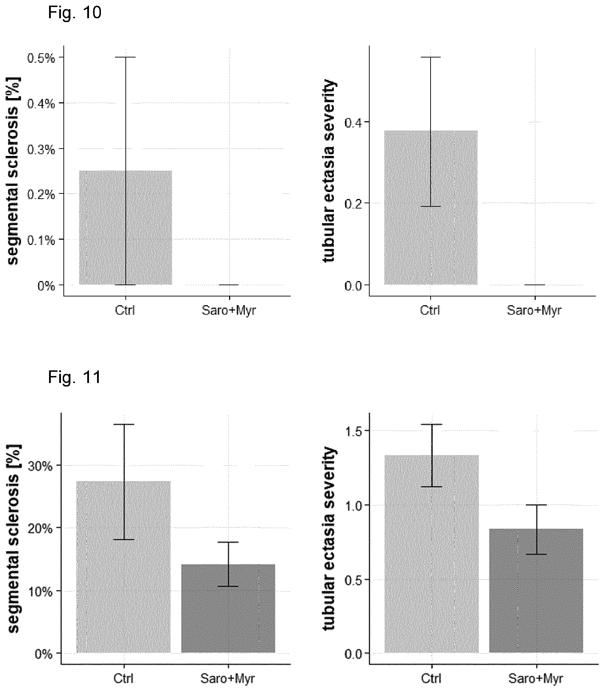

Fig. 10 graphically displays the impact of Saro+Myr therapy on the histopathology in the adriamycin nephropathy model (Exp. 2).

Fig. 11 graphically displays the impact of Saro+Myr therapy on the histopathology in the miR-193a FSGS model (Exp. 3).

Importantly, Saro+Myr therapy completely prevented severe FSGS on the histopathological level; in none of the adriamycin nephropathy samples a pathologic lesion was found. In the miR-193a model, which causes a much stronger disease, none of the samples had more than 25% diseased glomeruli or a tubular ectasia stage higher than 1.

Proteinuria is widely regarded as primary biomarker to monitor treatment in FSGS, as proteinuria reduction is considered to slow the progressive course of FSGS. As a next step in clinical development, an exploratory clinical trial in FSGS patients will repeatedly assess kidney function in timed urine specimens for proteinuria and creatinine excretion to assess the UPCR, as well as in blood for serum creatinine to assess the estimated Glomerular Filtration Rate (eGFR).
In summary, Saro+Myr treatment strongly and significantly improved proteinuria/UPCR, the central read-out parameter for FSGS in two different models, and also ameliorated histopathological lesions.

### REFERENCES

Cayla G. et al., Flow cytometric assessment of vasodilator-stimulated phosphoprotein: prognostic value of recurrent cardiovascular events after acute coronary syndromes, 2008, 101(11-12), 743-51
Chen A. et al., Soluble RARRES1 induces podocyte apoptosis to promote glomerular disease progression, 2020, J.Clin.lnvest, 130(10), 5523-5535
Chung CF. et al., Intrinsic tumor necrosis factor-α pathway is activated in a subset of patients with focal segmental glomerulosclerosis, 2019, PLoS One, 14(5):e0216426
Gebeshuber CA, et al. Focal segmental glomerulosclerosis is induced by microRNA-193a and its downregulation of WT1, 2013, Nat Med., 19(4),481-487
Harris J.J. et al., Active proteases in nephrotic plasma lead to a podocin-dependent phosphorylation of VASP in podocytes via protease activated receptor-1, 2013, J Pathol., 229(5), 660-71
Hassan NF, et al., Saroglitazar Deactivates the Hepatic LPS/TLR4 Signaling Pathway and Ameliorates Adipocyte Dysfunction in Rats with High-Fat Emulsion/LPS Model-Induced Non-alcoholic Steatohepatitis, 2019, Inflammation, 42(3), 1056-1070.
Jain, M.R., et al., Saroglitazar, a Novel PPARα/γ Agonist with Predominant PPARα Activity, Shows Lipid-Lowering and Insulin-Sensitizing Effects in Preclinical Models, 2015, Pharmacology Research & Perspectives, 3 (3), e00136. https://doi.org/10.1002/prp2.136
Kanjanabuch T, et al., PPAR-gamma agonist protects podocytes from injury., 2007, Kidney International, 71(12), 1232-1239.
Lloyd V.A. Jr., 2016, The Art, Science, and Technology of Pharmaceutical Compounding, 5th Edition, elSBN: 1-58212-263-6
Makled MN, El-Kashef DH. Saroglitazar attenuates renal fibrosis induced by unilateral ureteral obstruction via inhibiting TGF-β/Smad signaling pathway, 2020, Life Sci., 253, 117729
Monserrat AJ, et al., Protective effect of myristic acid on renal necrosis occurring in rats fed a methyl-deficient diet., 2000, Research in Experimental Medicine, Berlin, 199(4), 195-206.Pedigo C.E. et al., Local TNF causes NFATc1-dependent cholesterol-mediated podocyte injury, 2016, J. Clin. Invest, 126(9), 3336-50
Rogacka D. et al., Regulation of podocytes function by AMP-activated protein kinase, 2020, Arch Biochem Biophys, 692, 108541
Rosenberg A.Z., Kopp J.B., Focal Segmental Glomerulosclerosis., Clin J Am Soc Nephrol., 2017, 12(3), 502-517.
Sakata T. et al., Antiplatelet therapy effectively reduces plasma plasminogen activator inhibitor-1 levels, 2011, Artherosclerosis, 214(2), 490-1
Siller-Matula J.M. et al., Multiple electrode aggregometry predicts stent thrombosis better than the vasodilator-stimulated phosphoprotein phosphorylation assay, 2010, 8(2), 351-9
Takato, T. et al., Chronic Administration of Myristic Acid Improves Hyperglycaemia in the Nagoya-Shibata-Yasuda Mouse Model of Congenital Type 2 Diabetes, 2017, Diabetologia, 60 (10), 2076-83. https://doi.org/10.1007/s00125-017-4366-4.
Tsai PY. et al., Antroquinonol reduces oxidative stress by enhancing the Nrf2 signaling pathway and inhibits inflammation and sclerosis in focal segmental glomerulosclerosis mice, 2011, Free Radic Biol Med, 50(11), 1503-16
Troyanov S, et al.; Toronto Glomerulonephritis Registry Group. Focal and segmental glomerulosclerosis: definition and relevance of a partial remission, 2005, J Am Soc Nephrol., 16(4),1061-1068
Udenwobele D.I. et al., 2017, Myristoylation: An Important Protein Modification in the Immune Response, Frontiers in Immunology, 8, 751, pp 1-16

## Claims

1. Pharmaceutical preparation comprising saroglitazar and myristic acid, or a salt or solvate thereof, for use in the treatment of a non-diabetic subject suffering from focal segmental glomerulosclerosis (FSGS).

2. The pharmaceutical preparation for use according to claim 1, wherein FSGS is a recurrent FSGS, primary FSGS or native FSGS, specifically it is a glomerulopathy **characterized by** any one of heavy proteinuria, nephrotic syndrome, glomerulonephritis, membranoproliferative glomerulonephritis, IgA glomerulonephritis, progressive renal failure, or glomerular lesions on histopathology.

3. The pharmaceutical preparation for use according to claim 1 or 2, wherein the pharmaceutical preparation is a medicinal product or a drug product, comprising saroglitazar and myristic acid, and a pharmaceutically acceptable carrier.

4. The pharmaceutical preparation for use according to any one of claims 1 to 3, wherein the preparation is administered systemically.

5. The pharmaceutical preparation for use according to any one of claims 1 to 4, wherein saroglitazar comprises the structure

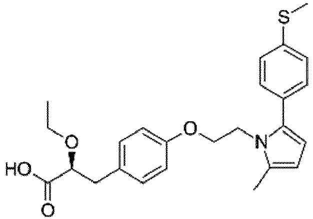

6. The pharmaceutical preparation for use according to claim 5, wherein the preparation comprises about 1 mg to 100 mg saroglitazar and 1 mg to 1 g myristic acid.

7. The pharmaceutical preparation for use according to any one of claims 1 to 6, wherein said pharmaceutical preparation is formulated for systemic administration, preferably for intravenous, intramuscular, subcutaneous, intradermal, transdermal, or oral administration.

8. The pharmaceutical preparation for use according to any one of claims 1 to 7, wherein said pharmaceutical preparation is administered to the subject as a spray, a powder, a gel, an ointment, a cream, a foam, or a liquid solution, a lotion, a gargle solution, an aerosolized powder, an aerosolized liquid formulation, granules, capsules, drops, tablet, syrup, lozenge, or a preparation for infusion or injection.

9. The pharmaceutical preparation for use according to any one of claims 1 to 8, wherein said pharmaceutical preparation is applied in an effective amount into a subject suffering from FSGS or being at risk of developing FSGS.

10. The pharmaceutical preparation for use according to any one of claims 1 to 9, wherein the preparation is administered as the sole substance, or wherein treatment is combined with a further treatment with one or more active substances.

11. The pharmaceutical preparation for use according to any one of claims 1 to 10, wherein the preparation is administered in combination with an active agent selected from the group consisting of antiviral drugs, anticoagulants, immune modulators, antibody preparations from human sources, monoclonal antibodies, intensive care medications, antihypertensive agents, statins, vasodilators, steroids, cytotoxic drugs, diuretics, non-steroidal anti-inflammatory drugs (NSAIDs), cholesterol or triglyceride reducing agents. PPAR agonists.

## Patentansprüche

1. Pharmazeutische Zubereitung umfassend Saroglitazar und Myristinsäure oder ein Salz oder Solvat davon zur Verwendung bei der Behandlung eines nichtdiabetischen Subjekts, das unter fokal-segmentaler Glomerulosklerose (FSGS) leidet.

2. Pharmazeutische Zubereitung zur Verwendung nach Anspruch 1, wobei die FSGS eine rezidivierende FSGS, primäre FSGS oder native FSGS ist, im Speziellen eine Glomerulopathie, die durch eine schwere Proteinurie, ein nephrotisches Syndrom, eine Glomerulonephritis, eine membranoproliferative Glomerulonephritis, eine IgA-Glomerulonephritis, ein progressives Nierenversagen oder glomeruläre Läsionen in der Histopathologie gekennzeichnet ist.

3. Pharmazeutische Zubereitung zur Verwendung nach Anspruch 1 oder 2, wobei die pharmazeutische Zubereitung ein medizinisches Produkt oder ein Arzneimittelprodukt ist, das Saroglitazar und Myristinsäure und einen pharmazeutisch akzeptablen Träger umfasst.

4. Pharmazeutische Zubereitung zur Verwendung nach einem der Ansprüche 1 bis 3, wobei die Zubereitung systemisch verabreicht wird.

5. Pharmazeutische Zubereitung zur Verwendung nach einem der Ansprüche 1 bis 4, wobei Saroglitazar die Struktur 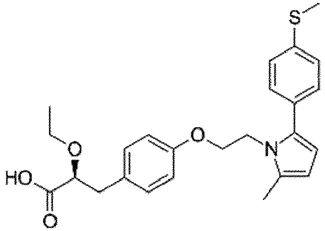 umfasst.

6. Pharmazeutische Zubereitung zur Verwendung nach Anspruch 5, wobei die Zubereitung etwa 1 mg bis 100 mg Saroglitazar und 1 mg bis 1 g Myristinsäure umfasst.

7. Pharmazeutische Zubereitung zur Verwendung nach einem der Ansprüche 1 bis 6, wobei die pharmazeutische Zubereitung für die systemische Verabreichung formuliert ist, vorzugsweise für die intravenöse, intramuskuläre, subkutane, intradermale, transdermale oder orale Verabreichung.

8. Pharmazeutische Zubereitung zur Verwendung nach einem der Ansprüche 1 bis 7, wobei die pharmazeutische Zubereitung an das Subjekt als ein Spray, ein Pulver, ein Gel, eine Salbe, eine Creme, ein Schaum oder eine flüssige Lösung, eine Lotion, eine Gurgellösung, ein zerstäubtes Pulver, eine zerstäubte flüssige Formulierung, als Granulat, Kapseln, Drops, Tablette, Sirup, Lutschtablette oder eine Zubereitung zur Infusion oder Injektion verabreicht wird.

9. Pharmazeutische Zubereitung zur Verwendung nach einem der Ansprüche 1 bis 8, wobei die pharmazeutische Zubereitung in einer wirksamen Menge in einem Subjekt angewendet wird, das unter einer FSGS leidet, oder für welches das Risiko besteht, eine FSGS zu entwickeln.

10. Pharmazeutische Zubereitung zur Verwendung nach einem der Ansprüche 1 bis 9, wobei die Zubereitung als einzige Substanz verabreicht wird, oder wobei die Behandlung mit einer weiteren Behandlung mit einer oder mehreren aktiven Substanzen kombiniert wird.

11. Pharmazeutische Zubereitung zur Verwendung nach einem der Ansprüche 1 bis 10, wobei die Zubereitung in Kombination mit einem aktiven Wirkstoff verabreicht wird, der aus der Gruppe ausgewählt ist, die aus antiviralen Arzneimitteln, Antikoagulanzien, Immun-Modulatoren, Antikörperzubereitungen aus menschlichen Quellen, monoklonalen Antikörpern, intensivmedizinischen Medikationen, antihypertensiven Mitteln, Statinen, Vasodilatoren, Steroiden, zytotoxischen Arzneimitteln, Diuretika, nichtsteroidalen antiinflammatorischen Arzneimitteln (NSAIDs), cholesterin- oder triglyceridsenkenden Mitteln und PPAR-Agonisten besteht.

## Revendications

1. Préparation pharmaceutique comprenant du saroglitazar et de l'acide myristique, ou un sel ou solvate de ceux-ci, destinée à être utilisée dans le traitement d'un sujet non diabétique souffrant de glomérulosclérose segmentaire focale (FSGS).

2. Préparation pharmaceutique destinée à être utilisée selon la revendication 1, dans laquelle la FSGS est une FSGS récidivante, une FSGS primaire ou une FSGS native, plus particulièrement celle-ci est une glomérulopathie **caractérisée par** l'un quelconque parmi une protéinurie élevée, le syndrome néphrotique, la glomérulonéphrite, la glomérulonéphrite membranoproliférative, la glomérulonéphrite à IgA, une insuffisance rénale progressive ou les lésions glomérulaires sur l'histopathologie.

3. Préparation pharmaceutique destinée à être utilisée selon la revendication 1 ou 2, la préparation pharmaceutique étant un produit médicinal ou un produit médicamenteux, comprenant du saroglitazar et de l'acide myristique et un vecteur pharmaceutiquement acceptable.

4. Préparation pharmaceutique destinée à être utilisée selon l'une quelconque des revendications 1 à 3, la préparation étant administrée de manière systémique.

5. Préparation pharmaceutique destinée à être utilisée selon l'une quelconque des revendications 1 à 4, dans laquelle le saroglitazar comprend la structure

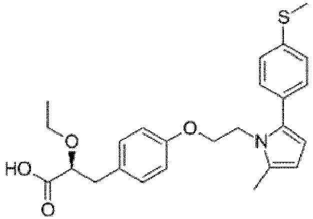

6. Préparation pharmaceutique destinée à être utilisée selon la revendication 5, la préparation comprenant environ 1 mg à 100 mg de saroglitazar et 1 mg à 1 g d'acide myristique.

7. Préparation pharmaceutique destinée à être utilisée selon l'une quelconque des revendications 1 à 6, ladite préparation pharmaceutique étant formulée pour une administration systémique, de préférence pour une administration intraveineuse, intramusculaire, sous-cutanée, intradermique, transdermique ou orale.

8. Préparation pharmaceutique destinée à être utilisée selon l'une quelconque des revendications 1 à 7, ladite préparation pharmaceutique étant administrée au sujet sous la forme d'un spray, d'une poudre, d'un gel, d'une pommade, d'une crème, d'une mousse, ou d'une solution liquide, d'une lotion, d'une solution de gargarisme, d'une poudre aérosolisée, d'une formulation liquide aérosolisée, de granulés, de capsules, de gouttes, d'un comprimé, d'un sirop, d'une pastille, ou d'une préparation pour perfusion ou injection.

9. Préparation pharmaceutique destinée à être utilisée selon l'une quelconque des revendications 1 à 8, ladite préparation pharmaceutique étant appliquée en une quantité efficace dans un sujet souffrant de FSGS ou présentant un risque de développer une FSGS.

10. Préparation pharmaceutique destinée à être utilisée selon l'une quelconque des revendications 1 à 9, la préparation étant administrée sous la forme d'une seule substance, ou la préparation étant combinée à un autre traitement avec un ou plusieurs principes actifs.

11. Préparation pharmaceutique destinée à être utilisée selon l'une quelconque des revendications 1 à 10, la préparation étant administrée en combinaison avec un principe actif choisi dans le groupe constitué par les médicaments antiviraux, les anticoagulants, les modulateurs immunitaires, les préparations d'anticorps provenant de sources humaines, les anticorps monoclonaux, les médicaments de soins intensifs, les agents antihypertenseurs, les statines, les vasodilatateurs, les stéroïdes, les médicaments cytotoxiques, les diurétiques, les médicaments anti-inflammatoires non stéroïdiens (NSAID), les agents réducteurs de cholestérol ou de triglycéride, et les agonistes de PPAR.
